# EUROPÄISCHE PATENTSCHRIFT

(11) **EP 1 928 459 B1**
(45) Veröffentlichungstag und Bekanntmachung des Hinweises auf die Patenterteilung: **18.08.2010**
(21) Anmeldenummer: 06722699.3
(22) Anmeldetag: 23.03.2006
(51) Int. Cl.: A61K 31/495, A61K 31/522, A61K 9/22

(54) **TABLETTENFÖRMIGE RETARDZUBEREITUNG ENTHALTEND CINNARIZIN UND DIMENHYDRINAT GEGEN SCHWINDEL**
TABLET-TYPE CONTROLLED-RELEASE PREPARATION CONTAINING CINNARIZINE AND DIMENHYDRINATE FOR COMBATING VERTIGO
PREPARATION A LIBERATION CONTROLEE SOUS FORME DE COMPRIME CONTENANT DE LA CINNARIZINE ET DU DIMENHYDRINATE CONTRE LE VERTIGE

(30) Priorität: 23.03.2005 DE 102005014141
(43) Veröffentlichungstag der Anmeldung: 11.06.2008
(73) Patentinhaber: Hennig Arzneimittel GmbH & Co. KG, 65439 Flörsheim (DE)
(72) Erfinder: FRANCAS, Gernot, 67549 Worms (DE)
(74) Vertreter: Schubert, Klemens
(86) Internationale Anmeldenummer: PCT/DE2006/000547
(87) Internationale Veröffentlichungsnummer: WO 2006/099865

(56) Entgegenhaltungen:
- WO-A-2004/064843
- HALAMA P: "ERFAHRUNGEN BEI DER THERAPIE DES VESTIBULAEREN UND ZEREBRALEN SCHWINDELS MIT ARLEVERT THERAPIEKONTROLLE MITTELS KRANIOKORPOGRAPHIE TREATMENT OF VESTIBULAR AND CEREBRAL VERTIGO WITH CINNARIZINE PLUS DIMENHYDRINATE" THERAPIEWOCHE, KARLSRUHE, DE, Bd. 35, Nr. 12, 1985, Seiten 1422-1426, XP008036742 ISSN: 0040-5973
- "SCHWINDELTHERAPIE MIT CINNARIZIN UND DIMENHYDRINAT KOMBINATIONSBEHANDLUNG IST EFFEKTIVER THERAPY OF VERTIGO WITH CINNARIZINE AND DIMENHYDRINATE: COMBINATION TREATMENT IS MORE EFFECTIVE" TW NEUROLOGIE, PSYCHIATRIE, BRAUN, KARLSRUHE, DE, Bd. 11, Nr. 12, 1997, Seiten 927-928, XP008036746 ISSN: 0935-3224

## Beschreibung

Die Erfindung betrifft eine pharmazeutische Zusammensetzung einer Retardzubereitung in Form von Tabletten zur Behandlung von Schwindel jedweder Genese.

Neben dem Kopfschmerz ist Schwindel (lat. vertigo) das häufigste von Patienten geklagte Symptom. Es handelt sich dabei um ein so genanntes multisensorisches Syndrom, das durch eine gestörte Wahrnehmung verschiedener Sinne gekennzeichnet ist und mit dem Verlust der Körpersicherheit im Raum und dadurch hervorgerufenen Gleichgewichtsstörungen einhergeht. In voller Ausprägung äußert sich Schwindel in der Wahrnehmung von Scheinbewegungen, in einer Fallneigung sowie in Übelkeit und Erbrechen. Schwindel kann sowohl episodisch als auch andauernd auftreten.

Unter Schwindel wird eine unangenehme Verzerrung der Raum- und Bewegungswahrnehmung verstanden, die zu Gleichgewichtsstörungen führt. Es handelt sich dabei nicht um eine eigenständige Krankheit, sondern um einen Symptomenkomplex unterschiedlicher Ursache und Herkunft, in den verschiedene Körpersinne involviert sind.

Aus der DE 103 01 981 A1 ist ein Präparat gegen Schwindel bekannt, welches Cinnarizin und Dimenhydrinat in Kombination enthält. Dabei wurde überraschenderweise gefunden, dass die Kombination der Wirkstoffe zu einem synergistischen Effekt führt.

Nachteilig ist hierbei jedoch, dass die Wirkung nach einiger Zeit nachlässt und es notwendig ist, mehrfach am Tage weitere Dosen zu applizieren. Bisher müssen diese Arzneimittel über den Tag verteilt bis zu 5 mal angewendet werden. Die damit einhergehenden Probleme in Bezug auf die Compliance sind somit klar.

Aufgabe der vorliegenden Erfindung ist es ein System zur Behandlung von Schwindel zur Verfügung zu stellen, dessen Wirkungsdauer gegenüber herkömmlichen Arzneimitteln verlängert ist.

Die Aufgabe der vorliegenden Erfindung wird durch eine pharmazeutische Zusammensetzung gemäß dem Hauptanspruch gelöst. Vorteilhafte Weiterbildungen der erfindungsgemäßen pharmazeutischen Zusammensetzung sind in den abhängigen Unteransprüchen gekennzeichnet.

Die Aufgabe wird erfindungsgemäß durch eine pharmazeutische Zusammensetzung enthaltend Cinnarizin und Dimenhydrinat gelöst, wobei die Wirkstofffreisetzung retardiert ist, diese erfindungsgemäße pharmazeutische Zusammensetzung weiterhin Bindemittel, Retardierungsmittel und Füllstoffe enthält,

wobei das Gewichts-Verhältnis von Bindemittel : Retardierungsmittel : Füllstoff zwischen 1 : 0,5 : 6 und 1 : 10 : 50 liegt. Besonders bevorzugt ist es, dass das Gewichts-Verhältnis von Bindemittel /Retardierungsmittel / Füllstoff zwischen 1 : 0,75 : 8,94 und 1 : 8 : 38,66 liegt.

Bevorzugt ist dabei ferner, dass das Bindemittel aus niedrigviskoser Hydroxypropylmethylcellulose (HPMC) ≤ 1000 cp, Hydroxypropylcellulose (HPG), Polyvinylpyrrolidon (PVP). Polyvinylacetat (PVA), Gelatine, und/oder Polysacchariden wie Gummi arabicum und Traganth oder aus deren Mischungen ausgewählt ist.

Bevorzugt ist dabei auch, dass das Retardierungsmittel aus hochviskoser Hydroxypropylmethylcellulose (HPMC) ≥ 1000 cp, und / oder Polysacchariden wie Alginsäure / Na-Alginat und Xanthangummi oder deren Mischungen ausgewählt ist.

Weiterhin ist dabei bevorzugt, dass der Füllstoff aus Lactose, Dextrose, Zucker, Sorbit, Mannit und / oder Stärke bzw. Stärkederivaten wie Na- Carboxymethylstärke oder deren Mischungen ausgewählt ist.

Bevorzugt ist ferner, dass in der erfindungsgemäßen pharmazeutischen Zusammensetzung weitere Hilfsstoffe enthalten sind.

Ein weiterer Gegenstand der vorliegenden Erfindung ist auch die Verwendung von Cinnarizin und Dimenhydrinat oder deren physiologisch verträglichen Salzen in Kombination zur Herstellung einer Arzneiform zur Behandlung von Schwindel jedweder Genese.

Cinnarizin (CAS 293-57-7) ist der internationale Freiname (INN) für 1-Benzhydryl-4-trans-cinnamylpiperazin. Es handelt sich hierbei um ein Antiverginosum, das nach neuesten Erkenntnissen hauptsächlich als Kalziumkanalblocker an den vestibulären Haarzellen wirkt. Dimenhydrinat (CAS 523-87-5) ist der internationale Freiname (INN) für das 8-Chlortheophyllin-Salz des Diphenhydramins und ist ein Antihistaminikum mit anticholinergen Eigenschaften, das antivertiginös und antiemetisch wirkt. Die Löslichkeiten der Wirkstoffe in Wasser sind sehr unterschiedlich, nämlich Cinnarizin ca. 2 mg / 100 ml, Dimenhydrinat ca. 3 mg / ml.

Aufgrund des stark unterschiedlichen Löslichkeitsverhaltens ist es bisher nicht gelungen, eine Retardform zu schaffen, welche die Wirkstoffe in derart synchroner Weise freisetzt, dass der synergistische Effekt der Wirkstoffkombination erhalten bleibt.

Überraschenderweise wurde nun gefunden, dass eine Kombination bestimmter Hilfsstoffe, sofern diese in einem bestimmten Mengenverhältnis nebeneinander vorliegen, den erfindungsgemäßen retardierenden Effekt aufweisen.

Es konnte gezeigt werden, dass Bindemittel, Retardierungsmittel und Füllstoffe in einem bestimmten Verhältnis nebeneinander vorliegen müssen, um den erfinderischen Effekt der zeitnahen Freisetzung der Wirkstoffe zu bewirken.

Die Aufgabe der Erfindung wird durch eine pharmazeutische Zusammensetzung gelöst, welche im Hauptanspruch beschrieben ist. Vorteilhafte Ausgestaltungen der erfindungsgemäßen Zusammensetzung sind in den abhängigen Unteransprüchen gekennzeichnet.

Die Aufgabe wird somit durch die Schaffung einer retardierenden Zusammensetzung gelöst, welche Bindemittel, Retardierungsmittel und Füllstoffe in einem definierten Gewichtsverhältnis enthalten. Dieses erfindungsgemäße Gewichts-Verhältnis von Bindemittel : Retardierungsmittel : Füllstoff liegt zwischen 1 : 0,5 : 6 und 1 : 10 : 50, besonders bevorzugt zwischen 1 : 0,75 : 8,94 und 1 : 8 : 38,66.

Überraschender Weise wurde nämlich gefunden, dass in diesem Mengenverhältnis die Freisetzung der Wirkstoffe Dimenhydrinat und Cinnarizin im optimalen Verhältnis zueinander erfolgt. Nur so kann der synergistische Effekt der Kombination dieser Wirkstoffe erzielt werden.

Erfindungsgemäße pharmazeutische Zusammensetzung enthalten mindestens ein Bindemittel, welches aus niedrigviskoser Hydroxypropylmethylcellulose (HPMC) ≤ 1000 cp, Hydroxypropylcellulose (HPC), Polyvinylpyrrolidon (PVP), Polyvinylacetat (PVA), Gelatine, und/oder Polysacchariden wie Gummi arabicum und Traganth oder aus deren Mischungen ausgewählt ist. Allerdings können auch andere, dem Fachmann als gleichwertig bekannte Bindemittel erfindungsgemäß verwendet werden. Es ist auch möglich, die genannten und andere Bindemittel zu kombinieren, um zu erfindungsgemäßen Zusammensetzungen zu gelangen.

Erfindungsgemäße pharmazeutische Zusammensetzung enthalten ferner mindestens ein Retardierungsmittel, welches aus hochviskoser Hydroxypropylmethylcellulose (HPMC) ≥ 1000 cp, und / oder Polysacchariden wie Alginsäure / Na-Alginat und Xanthangummi oder deren Mischungen ausgewählt ist. Allerdings können auch andere, dem Fachmann als gleichwertig bekannte Retardierungsmittel erfindungsgemäß verwendet werden. Es ist auch möglich, die genannten und andere Retardierungsmittel zu kombinieren, um zu erfindungsgemäßen Zusammensetzungen zu gelangen.

Erfindungsgemäße pharmazeutische Zusammensetzung enthalten ferner mindestens einen Füllstoff, welcher aus Lactose, Dextrose, Zucker, Sorbit, Mannit und / oder Stärke bzw. Stärkederivaten wie Na- Carboxymethylstärke oder deren Mischungen ausgewählt ist. Allerdings können auch andere, dem Fachmann als gleichwertig bekannte Füllstoffe erfindungsgemäß verwendet werden. Es ist auch möglich, die genannten und andere Füllstoffe zu kombinieren, um zu erfindungsgemäßen Zusammensetzungen zu gelangen.

Weiterhin können in den erfindungsgemäßen pharmazeutischen Zusammensetzungen weitere Hilfsstoffe enthalten sein. Derartige Hilfsstoffe sind dem Fachmann bekannt und werden zugesetzt, um die gewünschten Arzneiformen herstellen zu können. Derartige Hilfsstoffe sind beispielsweise Magnesiumstearat, Talkum, Aerosil, Trennmittel, Gleitmittel und dergleichen, welche die Verarbeitung der Mischungen mit Maschinen erleichtert und/oder ermöglicht.

Erfindungsgemäß geeignete Zubereitungsformen sind dem Fachmann bekannt und können Tabletten, Filmtabletten und andere Formen sein.

Bevorzugt ist es auch, wenn die Tabletten mit einem Lack als Schluckhilfe versehen sind. Ein derartiger Lack ist dem Fachmann bekannt. Dieser Lack besteht aus einem Filmbildner wie Eudragit E, RL, RS oder HPMC, umfasst ferner Weichmacher wie PEG, Triacin oder Polysorbat, Pigmente wie TiO₂, Farbstoffe wie Eisenoxide und ggf. Trennmittel wie Talkum. Die Herstellung eines solchen Lacks, bzw. das Lackieren der fertigen Tabletten ist dem Fachmann bekannt.

Die Herstellung derartiger Arzneiformen ist dem Fachmann bekannt (e. g. Hagers Handbuch der Pharmazeut. Praxis, 5. Aufl. von 1990-1995 im Springer-Verlag, Berlin).

Die nachfolgenden Beispiele erläutern die Erfindung:

### Beispiel 1

### Allgemeines Herstellungsverfahren zur Herstellung der erfindungsgemäßen Retardzubereitungen

Die Herstellung der erfindungsgemäßen pharmazeutischen Zusammensetzungen erfolgt in an sich bekannter Weise. Dabei werden
in einem ersten Ansatz Dimenhydrinat, Cinnarizin, Bindemittel, Retardierungsmittel, und Füllstoffe vorgemischt, granuliert, gesiebt und getrocknet,
in einem zweiten Ansatz Dimenhydrinat, Cinnarizin, Bindemittel und Füllstoffe gemischt, gesiebt und homogenisiert,
und dann die Produkte aus dem ersten Ansatz und dem zweiten Ansatz durch Mischen homogenisiert, Magnesiumstearat hinzugegeben und nochmals gemischt. Die so erhaltene
Tablettiermischung wird dann zu geeigneten Tabletten verpresst.

Es wurden erfindungsgemäße Zusammensetzungen nach verschiedenen Rezepturen wie oben angegeben hergestellt und deren Freisetzung nach der Paddle-Methode gemäß der Europäischen Pharmäkopöe bestimmt.

### Beispiele 2 bis 11

Folgende erfindungsgemäße Zusammensetzungen gemäß den Rezepturen 1 bis 10 wurden hergestellt:

**Tabelle 1**

| | **Rezepturen** | | | | | | | | | |
|---|---|---|---|---|---|---|---|---|---|---|
| **Bestandteil** | **1** | **2** | **3** | **4** | **5** | **6** | **7** | **8** | **9** | **10** |
| **Wirkstoff** | | | | | | | | | | |
| Dimenhydrinat | 120 | 120 | 120 | 120 | 120 | 120 | 120 | 120 | 120 | 120 |
| Cinnarizin | 60 | 60 | 60 | 60 | 60 | 60 | 60 | 60 | 60 | 60 |

| **Bindemittel** | | | | | | | | | | |
|---|---|---|---|---|---|---|---|---|---|---|
| HPMC niedrigviskos | | | | | | | 5 | 10 | 14 | 13,3 |
| HPC | | | | | | 5 | | | | |
| PVP | | | 5 | | | | | | | |
| PVA | 20 | | | | | | | | | |
| Gelatine | | 10 | | | | | | | | |
| Gummi arabicum | | | | | 10 | | | | | |
| Traganth | | | | 20 | | | | | | |

| **Retardierungsmittel** | | | | | | | | | | |
|---|---|---|---|---|---|---|---|---|---|---|
| HPMC hochviskos | | | | | | | 20 | 15 | 14 | 13,3 |
| Alginsäure | 20 | | | | | | | | | |
| Na-Alginat | | 30 | 40 | | | | | | | |
| Xanthangummi | | | | 15 | 25 | 35 | | | | |

| **Füllstoff** | | | | | | | | | | |
|---|---|---|---|---|---|---|---|---|---|---|
| Lactose | | | 103,6 | 144,1 | 153,4 | | | | 130 | 138,7 |
| Dextrose | 80 | 100 | | | | | 173,3 | 161,8 | | |
| Zucker | | | 70 | | | | | | | |
| Sorbit | | | | 40 | 30 | | | | | |
| Mannit | | | | | | 50 | | | | |
| Stärke | 98,8 | 79,3 | | | | 128,9 | | | 60 | 53;4 |
| Na-Carboxymethylstärke | | | | | | | 20 | 32 | | |

| **Sonstige** | | | | | | | | | | |
|---|---|---|---|---|---|---|---|---|---|---|
| Magnesiumstearat | 0,7 | 0,7 | 0,9 | 0,9 | 1,1 | 1,1 | 1,2 | 1,2 | 1,3 | 1,3 |
| Aerosil | 0,5 | | 0,5 | | 0,5 | | 0,5 | | 0,7 | |
| **Tablettengewicht** | 400 mg | | | | | | | | | |

Im folgenden wird die Erfindung anhand von Tabellen und Figuren näher erläutert.

Im Einzelnen zeigt:
- Tabelle 2: eine tabellarische Darstellung der Freisetzung der erfindungsgemäßen pharmazeutischen Zusam- mensetzung gemäß Rezeptur 10 aus Tabelle 1
- Figur 1: ein Diagramm der Freisetzung von Cinnarizin,
- Figur 2: ein Diagramm der Freisetzung von Dimenhydrinat,
- Tabelle 3: eine tabellarische Darstellung der Freisetzung einer herkömmlichen Cinnarizin/Dimenhydrinat- Zubereitung,
- Figur 3: ein Diagramm der Freisetzung von Cinnarizin und
- Figur 4: ein Diagramm der Freisetzung von Dimenhydrinat.

Die Tabelle 2 zeigt eine tabellarische Darstellung der Freisetzung von.Cinnariazin und Dimenhydrat bei Verwendung der erfindungsgemäßen pharmazeutischen Zusammensetzung gemäß Rezeptur 10 der Tabelle 1. Es wurden drei verschiedene Chargen (03/675, 03/676 und 03/677) geprüft.

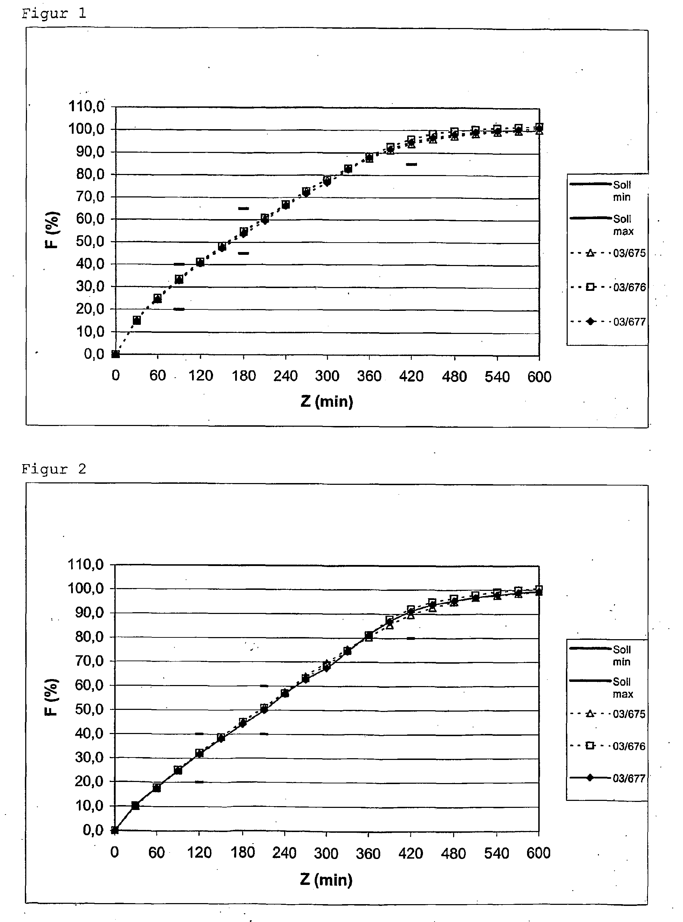

Ein Diagram der Freisetzung von Cinnarizin (W1) aus der erfindungsgemäßen Zusammensetzung ist in Figur 1 dargestellt.

Figur 2 zeigt ein Diagramm in dem die Freisetzung (F) von Dimenhydrinat (W2) in Abhängigkeit von der Zeit (Z) aufgetragen ist.

Im Vergleich zu Tabelle 2 zeigt die Tabelle 3 eine tabellarische Darstellung der Freisetzung von Cinnarizin und Dimenhydrat bei einer herkömmlichen, nicht erfindungsgemäßen Cinnariazin/Dimenhydrat-Zubereitung.

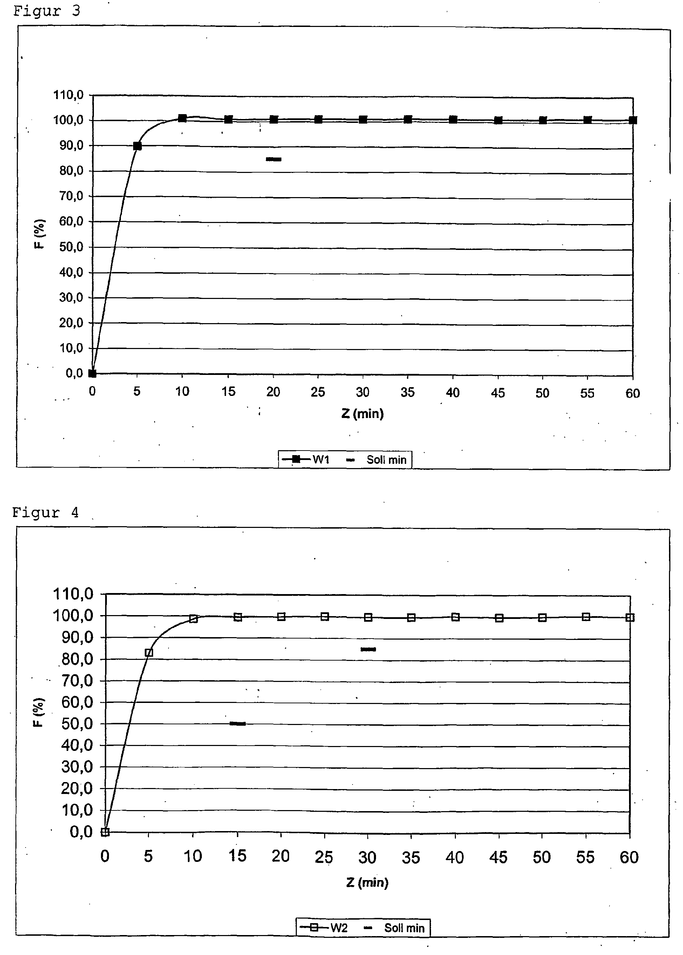

Die Figur 3 zeigt ein Diagramm in dem die Freisetzung (F) von Cinnarizin (W1) in Anhängigkeit von der Zeit (Z) aufgetragen ist.

Die Figur 4 zeigt ein Diagramm in dem die Freisetzung (F) von Dimenhydrinat (W2) in Abhängigkeit von der Zeit (Z) aufgetragen ist.

Aus der erfindungsgemäßen pharmazeutischen Zusammensetzung beträgt die In-vitro-Freisetzung der Cinnarizin Base nach 90 Minuten 20 % -40 %, nach 180 Minuten 45 % - 65 % und nach 420 Minuten > 85 %. Die In-vitro-Freisetzung von Dimenhydrinat beträgt nach 120 Minuten 20 % - 40 %, nach 210 Minuten 40 % - 60 % und nach 420 Minuten > 80 %.

Ein Vergleich der Figuren 1 und 3 sowie 2 und 4 zeigt, dass die Freisetzung von Cinnarizin bzw. Dimenhydrat bei der unretardierten Mischung von Cinnarizin und Dimenhydrat zunächst stark ansteigt und dann auf einem Wert von ca. 100 % bleibt, während der Anstieg der Freisetzung bei der erfindungsgemäßen Zusammensetzung verzögert stattfindet.

**Tabelle 2**

| **Zeit** | Freisetzungs- Mittelwerte (%) (F) | | | | | | | | | |
|---|---|---|---|---|---|---|---|---|---|---|
| **(Z)** | Cinnarizin (W1) | | | | | Dimenhydrinat (W2) | | | | |
| **(min)** | **Soll min** | **Soll max** | **03/675** | **03/676** | **03/677** | **Soll min** | **Soll max** | **03/675** | **03/676** | **03/677** |
| **0** | | | 0,0 | 0,0 | 0,0 | | | 0,0 | 0,0 | 0,0 |
| **30** | | | 14,9 | 15,3 | 15,0 | | | 9,9 | 10,2 | 10,3 |
| **60** | | | 24,6 | 25,1 | 24,5 | | | 17,5 | 17,9 | 17,8 |
| **90** | **20,0** | **40,0** | 33,2 | 33,6 | 32,9 | | | 24,9 | 25,2 | 24,9 |
| **120** | | | 41,0 | 41,3 | 40,4 | **20,0** | **40,0** | 32,0 | 32,2 | 31,7 |
| **150** | | | 48,1 | 48,4 | 47,3 | | | 38,8 | 38,8 | 38,1 |
| **180** | **45,0** | **65,0** | 54,6 | 54,9 | 53,7 | | | 45,2 | 45,0 | 44,1 |
| **210** | | | 60,6 | 60,9 | 59,6 | **40,0** | **60,0** | 51,2 | 50,9 | 49,9 |
| **240** | | | 66,8 | 66,9 | 66,3 | | | 57,5 | 57,0 | 56,8 |
| **270** | | | 72,9 | 72,7 | 71,5 | | | 64,1 | 63,2 | 62,7 |
| **300** | | | 78,0 | 78,0 | 76,6 | | | 69,7 | 68,8 | 67,6 |
| **330** | | | 83,1 | 83,0 | 82,5 | | | 75,4 | 74,7 | 74,4 |
| **360** | | | 87,4 | 88,4 | 87,9 | | | 80,3 | 81,2 | 81,3 |
| **390** | | | 91,2 | 92,8 | 91,7 | | | 85,5 | 87,9 | 87,0 |
| **420** | **85,0** | | 94,0 | 96,0 | 94,7 | **80,0** | | 89,5 | 92,0 | 91,1 |
| **450** | | | 96,0 | 98,4 | 96,7 | | | 92,6 | 94,9 | 93,8 |
| **480** | | | 97,4 | 99,6 | 98,1 | | | 95,1 | 96,6 | 95,5 |
| **510** | | | 98,5 | 100,4 | 99,2 | | | 96,8 | 97,9 | 96,8 |
| **540** | | | 99,3 | 101,1 | 99,9 | | | 97,7 | 99,1 | 97,9 |
| **570** | | | 99,9 | 101,6 | 100,5 | | | 98,4 | 99,9 | 98,7 |
| **600** | | | 100,3 | 102,0 | 101,3 | | | 99,3 | 100,5 | 99,4 |

**Tabelle 3**

| **Zeit** | Freisetzungs- Mittelwerte (%)(F) | | | |
|---|---|---|---|---|
| **(Z)** | Cinnarizin (W1) | | Dimenhydrinat (W2) | |
| **(min)** | **Soll min** | **03/633** | **Soll min** | **03/633** |
| **0** | | 0,0 | | 0,0 |
| **5** | | 89,9 | | 82,8 |
| **10** | | 100,9 | | 98,5 |
| **15** | | 100,8 | **50,0** | 99,5 |
| **20** | **85,0** | 100,9 | | 99,8 |
| **25** | | 101,0 | | 100,0 |
| **30** | | 100,9 | **85,0** | 99,7 |
| **35** | | 100,9 | | 99,8 |
| **40** | | 101,0 | | 100,0 |
| **45** | | 100,9 | | 99,7 |
| **50** | | 101,1 | | 99,9 |
| **55** | | 101,3 | | 100,3 |
| **60** | | 101,2 | | 100,1 |

## Patentansprüche

1. Pharmazeutische Zusammensetzung mit retardierter Wirkstofffreisetzung, enthaltend Cinnarizin und Dimenhydrinat, Bindemittel, Retardierungsmittel und Füllstoffe, **dadurch gekennzeichnet, dass** das Gewichts-verhältnis von Bindemittel / Retardierungsmittel / Füllstoff zwischen 1 : 0,5 : 6 und 1 : 10 : 50 liegt.

2. Pharmazeutische Zusammensetzung gemäß Anspruch 1, **dadurch gekennzeichnet, dass** das Gewichts-Verhältnis von Bindemittel / Retardierungsmittel / Füllstoff zwischen 1 : 0,75 : 8,94 und 1 : 8 : 38,66 liegt.

3. Pharmazeutische Zusammensetzung gemäß einem der voranstehenden Ansprüche, **dadurch gekennzeichnet, dass** das Bindemittel aus niedrigviskoser Hydroxypropylmethylcellulose (HPMC) ≤ 1000 cp, Hydroxypropylcellulose (HPC), Polyvinylpyrrolidon (PVP), Polyvinylacetat (PVA), Gelatine, und/oder Polysacchariden wie Gummi arabicum und Traganth oder aus deren Mischungen ausgewählt ist.

4. Pharmazeutische Zusammensetzung gemäß einem der voranstehenden Ansprüche, **dadurch gekennzeichnet, dass** das Retardierungsmittel aus hochviskoser Hydroxypropylmethylcellulose (HPMC) ≥ 1000 cp, und / oder Polysacchariden wie Alginsäure / Na-Alginat und Xanthangummi oder deren Mischungen ausgewählt ist.

5. Pharmazeutische Zusammensetzung gemäß einem der voranstehenden Ansprüche, **dadurch gekennzeichnet, dass** der Füllstoff aus Lactose, Dextrose, Zucker, Sorbit, Mannit und / oder Stärke bzw. Stärkederivaten wie Na-Carboxymethylstärke oder deren Mischungen ausgewählt ist.

6. Pharmazeutische Zusammensetzung gemäß einem der voranstehenden Ansprüche, **dadurch gekennzeichnet, dass** weitere Hilfsstoffe enthalten sind.

7. Pharmazeutische Zusammensetzung gemäß einem der voranstehenden Ansprüche, **dadurch gekennzeichnet, dass** diese in Form von Tabletten vorliegen und mit einem Lack überzegen sind.

8. Verwendung der pharmazeutischen Zusammensetzung nach einem der voranstehenden Ansprüche zur Herstellung einer Arzneiform zur Behandlung von Schwindel jedweder Genese.

## Claims

1. A pharmaceutical composition containing cinnarizine and dimenhydrinate, binding agent, slow-release agent and fillers, hereby **characterized in that** the release of active ingredients is slowed down and that the weight ratio of binding agent/slow-release agent/filler lies between 1:0.5:6 and 1:10:50.

2. The pharmaceutical composition as claimed in claim 1, further **charactarized in that** the weight ratio of binding agent/slow-release agent/filler lies between 1:0.75:8.94 and 1:8:38.66.

3. The pharmaceutical composition as claimed in one of the preceding claims, further **charactarized in that** the binding agent is selected from low-viscosity hydroxypropylmethylcellulose (HPMC) ≤ 1000 cp, hydroxypropylcellulose (HPC), polyvinylpyrrolidone (PVP), polyvinyl acetate (PVA), gelatin, and/or polysaccharides such as gum arabic und tragacanth or from their mixtures.

4. The pharmaceutical composition as claimed in one of the preceding claims, further **charactarized in that** the slow-release agent is selected from high-viscosity hydroxypropylmethylcellulose (HPMC) ≥ 1000 cp, and/or polysaccharides such as alginic acid/Na alginate and xanthan gum or their mixtures.

5. The pharmaceutical composition as claimed in one of the preceding claims, further **charactarized in that** the filler is selected from lactose, dextrose, sugar, sorbitol, mannitol and/or starch or starch derivatives such as Na carboxymethyl starch or their mixtures.

6. The pharmaceutical composition as claimed in one of the preceding claims, further **charactarized in that** additional auxiliary agents are contained in it.

7. The pharmaceutical composition as claimed in one of the preceding claims, further **charactarized in that** the composition is in form of tablets and the tablets are coated with a lacquer.

8. The use of the pharmaceutical composition according to one of the preceding claims for the production of a medicament for the treatment of vertigo of any genesis.

## Revendications

1. Composition pharmaceutique à libération de substance active retardée, comprenant de la cinnarizine et du diménhydrinate, des liants, des agents de retardement et des substances de charge, **caractérisée en ce que** le rapport pondéral liant/agent de retardement/substance de charge se situe entre 1 : 0,5 : 6 et 1 : 10 : 50.

2. Composition pharmaceutique selon la revendication 1, **caractérisée en ce que** le rapport pondéral liant/agent de retardement/substance de charge se situe entre 1 : 0,75 : 8,94 et 1 : 8 : 38,66.

3. Composition pharmaceutique selon l'une quelconque des revendications précédentes, **caractérisée en ce que** le liant est choisi parmi l'hydroxypropylméthylcellulose (HPMC) à faible viscosité ≤ 1000 cp, l'hydroxypropylcellulose (HPC), la polyvinylpyrrolidone (PVP), l'acétate de polyvinyle (PVA), la gélatine et/ou des polysaccharides tels que la gomme arabique et la gomme adragante, ou encore leurs mélanges.

4. Composition pharmaceutique selon l'une quelconque des revendications précédentes, **caractérisée en ce que** l'agent de retardement est choisi parmi l'hydroxypropylméthylcellulose (HPMC) à viscosité élevée ≥ 1000 cp, et/ou des polysaccharides tels que l'acide alginique / l'alginate de sodium et la gomme de xanthane ou leurs mélanges.

5. Composition pharmaceutique selon l'une quelconque des revendications précédentes, **caractérisée en ce que** la substance de charge est choisie parmi le lactose, le dextrose, le sucre, le sorbitol, le mannitol et/ou l'amidon respectivement des dérivés de l'amidon tels que le carboxyméthylamidon de sodium, ou leurs mélanges.

6. Composition pharmaceutique selon l'une quelconque des revendications précédentes, **caractérisée en ce qu'**elle contient des substances auxiliaires supplémentaires.

7. Composition pharmaceutique selon l'une quelconque des revendications précédentes, **caractérisée en ce qu'**elle est présente sous forme de comprimés et **en ce qu'**elles sont munis d'un enrobage avec une gomme laque.

8. Utilisation de la composition pharmaceutique selon l'une quelconque des revendications précédentes pour la préparation d'une forme médicamenteuse destinée au traitement du vertige quelle que soit son origine.
